# EUROPEAN PATENT SPECIFICATION

(11) **EP 0 382 526 B1**
(45) Date of publication and mention of the grant of the patent: **15.05.1996**
(21) Application number: 90301335.7
(22) Date of filing: 08.02.1990
(51) Int. Cl.: C07D 473/00, C07D 411/04, C07D 411/14, C07D 327/04, A61K 31/52, A61K 31/505

(54) **Substituted -1,3-oxathiolanes with antiviral properties**
Substituierte 1,3-Oxathiolane mit antiviraler Wirkung
1,3-oxathiolanes substitués doués de propriétés antivirales

(30) Priority: 08.02.1989 US 308101
(43) Date of publication of application: 16.08.1990
(60) Divisional application: 95120531.9
(73) Proprietor: BIOCHEM PHARMA INC., Laval, Quebec H7V 4A7 (CA)
(72) Inventor: Belleau, Bernard (deceased), / (CA); Belleau, Pierette, Westmont, Quebec H3Y 2R3 (CA); Nguyen-Ba, Nghe, Brossard, Quebec J42 1G6 (CA)
(74) Representative: Ritter, Stephen David

(56) References cited:
- EP-A- 0 206 497
- EP-A- 0 337 713
- WO-A-91/11186

## Description

The present invention relates to novel substituted 1,3-oxathiolane cyclic compounds having pharmacological activity, to processes for and intermediates of use in their preparation, to pharmaceutical compositions containing them, and to the use of these compounds in the antiviral treatment of mammals.

Retroviral infections are a serious cause of disease, most notably, the acquired immunodeficiency syndrome (AIDS). The human immunodeficiency virus (HIV) has been recognized as the etiologic agent of AIDS and compounds having an inhibitory effect against HIV multiplication have been actively sought.

Mitsuya et al., "3'-Azido-3'-deoxythymidine (BW A509U): An antiviral agent that inhibits the infectivity and cytopathic effect of human T-lymphotropic virus type III/lymphadenopathy-associated virus in vitro", Proc. Natl. Acad. Sci. U.S.A., 82, pp. 7096-7100 (1985), refers to a compound of formula (A) (3'-azido-2'3'-dideoxythymidine), commonly referred to as AZT. This compound is said to be useful in providing some protection for AIDS carriers against the cytopathogenic effect of immunodeficiency virus (HIV).

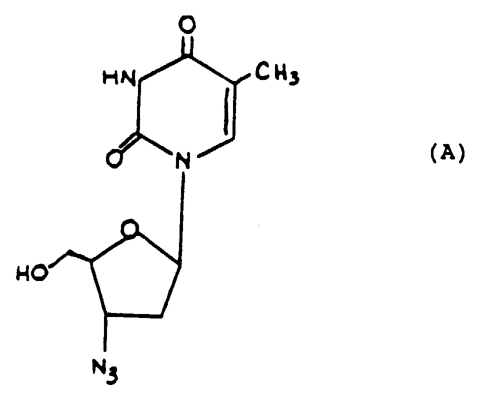

Mitsuya et al., "Inhibition of the in vitro infectivity and cytopathic effect of human T-lymphotrophic virus type III/lymphadenopathy-associated virus (HTLV-III/LAV) by 2'3'-dideoxynucleosides", Proc. Natl. Acad. Sci. U.S.A., 86, pp. 1911-15 (1986), have also referred to a group of 2',3'- dideoxynucleosides shown in formula (B) which are said to possess protective activity against HIV-induced cytopathogenicity.

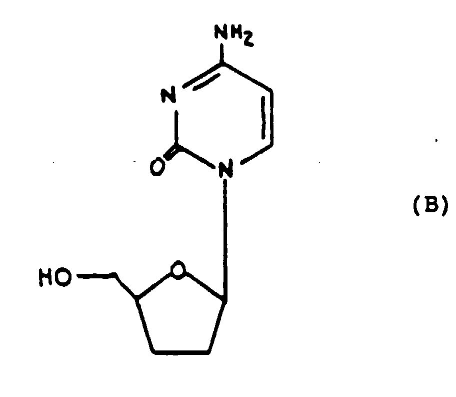

Balzarini et al., "Potent and selective anti-HTLV-III/LAV activity of 2',3'-dideoxycytidinene, the 2',3'-unsaturated derivative of 2',3'-dideoxycytidine", Biochem. Biophys. Res. Comm., 140, pp. 735-42 (1986), refer to an unsaturated analogue of these nucleosides--2'3'-dideoxy-cytidine, shown in formula (C)--as being characterized by antiretroviral activity.

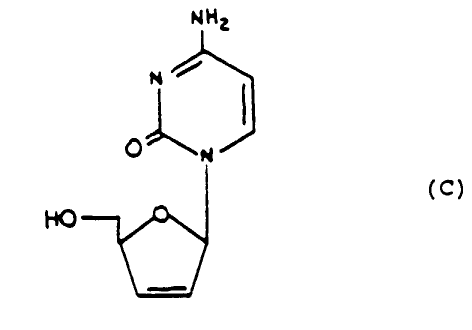

Baba et al., "Both 2',3'-dideoxythymidine and its 2',3'-unsaturated derivative (2',3'-dideoxythymidinene) are potent and selective inhibitors of human immunodeficiency virus replication in vitro", Biochem. Biophys. Res. Comm., 142, pp. 128-34 (1987), refer to the 2',3'-unsaturated analogue shown in formula (D) of 2',3'-dideoxythymidine. This analogue is purported to be a potent selective inhibitor of HIV replication.

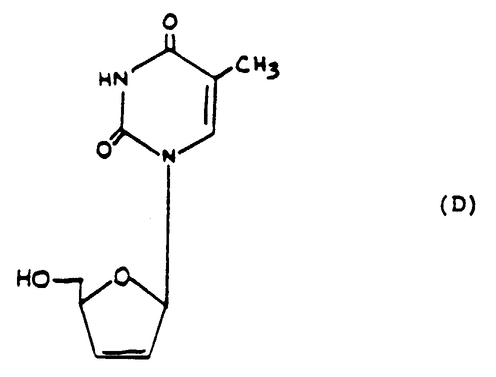

Analogues of AZT known as 3'-azido-2', 3'-dideoxyuridine shown in formula (E), where Y is bromine or iodine, have been said to have an inhibitory activity against Moloney murine leukemia in T.S. Lin et al., "synthesis and antiviral activity of various 3'-azido, 3' amino, 2',3'-unsaturated and 2',3'- dideoxy analogues of pyrimidine, deoxyribonucleosides against retroviruses", J. Med. Chem., 30, pp. 440-41 (1987).

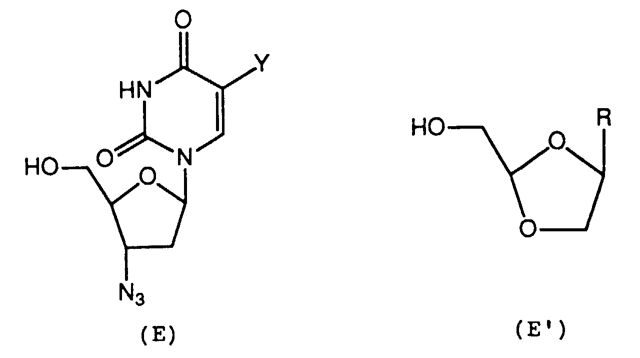

European publication EP A-337,713 is directed to 1,3-dioxolane nucleoside analogues of formula (E') with antiviral properties.

Finally, the 3'-fluoro analogues of 2',3'-dideoxycytidine shown in formula (F) and of 2',3'-dideoxythymidine shown in formula (G) are referred to in Herdewijn et al., "3'-Substituted 2',3'-dideoxynucleoside analogues as potential anti- HIV(HTLV-III/LAV) agents", J. Med. Chem., 30, pp. 1270-78 (1987), as having potent antiretroviral activity.

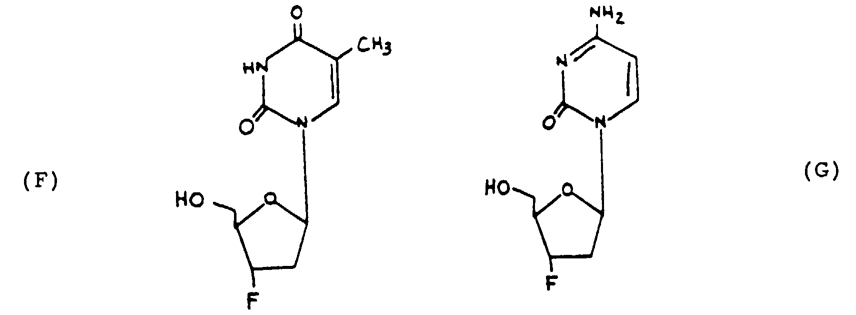

The most potent anti-HIV compounds thus far reported are 2',3'-dideoxynucleosides, more particularly, 2',3'-dideoxy cytidine (ddCyd) and 3'-azido-2',3'-dideoxythymidine (AzddThd or AZT). These compounds are also active against other kinds of retroviruses such as the Moloney murine leukemia virus. Because of the increasing incidence and the life-threatening characteristics of AIDS, efforts are being expended to discover and develop new non-toxic and potent inhibitors of HIV and blockers of its infectivity. It is therefore an object of the present invention to provide effective anti-HIV compounds of low toxicity and a synthesis of such new compounds that is readily feasible.

The present invention provides novel 2-substituted-5-substituted-1,3-oxathiolanes which have antiretroviral activity. In particular, these compounds have been found to act as non-toxic inhibitors of the replication of HIV-1 in T-lymphocytes over prolonged periods of time.

There is accordingly provided in a first aspect cis-2-hydroxymethyl-5-(cytosin-1'-yl)-1,3-oxathiolane, and pharmaceutically acceptable derivatives thereof.

Cis-2-hydroxymethyl-5-(cytosin-1'-yl)-1,3-oxathiolane may be represented by the formula

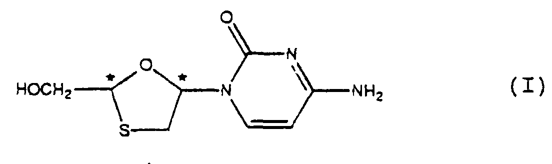

It will be appreciated by those skilled in the art that formula (I) contains two chiral centers (shown as * in formula (I)) and thus includes two pairs of optical isomers (i.e. enantiomers) and mixtures thereof including racemic mixtures. Both cis-isomers and mixtures thereof, including racemic mixtures, are included within the scope of the invention. The cis-isomers may also be represented by formula (II), and similarly, the trans-isomers may be represented by formula (III)

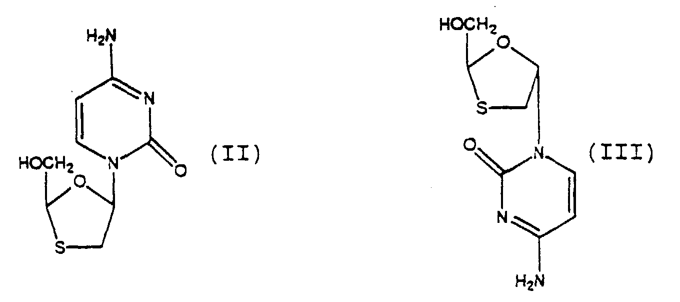

By "a pharmaceutically acceptable derivative" means any pharmaceutically acceptable salt of a compound of formulae (I) or (II), and the following esters thereof : a C₁₋₁₆ alkanoyl ester, an unsubstituted benzoyl ester or a benzoyl ester substituted by at least one halogen (bromine, chlorine, fluorine or iodine), C₁₋₆ alkyl, C₁₋₆ alkoxy, nitro or trifluoromethyl groups.

It will be appreciated by those skilled in the art that the compounds of formulae (I) or (II) may be modified to provide pharmaceutically acceptable esters at the hydroxymethyl group of the oxathiolane ring.

Preferred esters of the compounds of formulae (I) or (II) include the compounds in which H is replaced by a carboxyl function 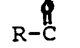 in which the non-carbonyl moiety R of the ester grouping is selected from hydrogen, straight or branched chain alkyl (e.g., methyl, ethyl, n-propyl, t-butyl, n-butyl), phenyl optionally substituted by halogen, C₁₋₄ alkyl or C₁₋₄ alkoxy.

Unless otherwise specified, any alkyl moiety present advantageously contains 1 to 16 carbon atoms, preferably 1 to 4 carbon atoms.

Pharmaceutically acceptable salts of the compounds of formulae (I) or (II) include those derived from pharmaceutically acceptable inorganic and organic acids and bases. Examples of suitable acids include hydrochloric, hydrobromic, sulfuric, nitric, perchloric, fumaric, maleic, phosphoric, glycollic, lactic, salicylic, succinic, toluene-p-sulfonic, tartaric, acetic, citric, methanesulfonic, formic, benzoic, malonic, naphthalene-2-sulfonic and benzenesulfonic acids. Other acids such as oxalic, while not in themselves pharmaceutically acceptable, may be useful in the preparation of salts useful as intermediates in obtaining the compounds of the invention and their pharmaceutically acceptable acid addition salts.

Salts derived from appropriate bases include alkali metal (e.g., sodium), alkaline earth metal (e.g., magnesium), ammonium and NR₄+ (where R is C₁₋₄ alkyl) salts.

References hereinafter to a compound according to the invention includes both compounds of formulae (I) or (II) and their pharmaceutically acceptable derivatives.

The compounds of the invention either themselves possess antiviral activity and/or are metabolizable to such compounds. In particular these compounds are effective in inhibiting the replication of retroviruses, including human retroviruses such as human immunodeficiency viruses (HIV's), the causative agents of AIDS.

There is thus provided as a further aspect of the invention a compound formula (I) or a pharmaceutically acceptable derivative thereof for use as an active therapeutic agent in particular as an antiviral agent, for example in the treatment of retroviral infections.

In a further or alternative aspect there is provided a method for the treatment of a viral infection, in particular an infection caused by a retrovirus such as HIV, in a mammal, including man, comprising administration of an effective amount of an antiviral compound of formula (I) or a pharmaceutically acceptable derivative thereof.

There is also provided in a further or alternative aspect of this invention, use of a compound of formula (I) or a pharmaceutically acceptable derivative thereof for the manufacture of a medicament for the treatment of a viral infection.

The compounds of the invention are also useful in the treatment of AIDS related conditions such as AIDS-related complex (ARC), persistent generalized lymphadenopathy (PGL), AIDS-related neurological conditions (such as dementia), anti-HIV antibody positive and HIV- positive conditions, Kaposi's sarcoma, thrombocytopenia purpurea and opportunistic infections.

The compounds of the invention are also useful in the prevention or progression to clinical illness of individuals who are anti-HIV antibody or HIV-antigen positive and in prophylaxis following exposure to HIV.

The compounds of formula (I) or the pharmaceutically acceptable derivatives thereof, may also be used for the prevention of viral contamination of biological fluids such as blood or semen in vitro.

It will be appreciated by those skilled in the art that references herein to treatment extends to prophylaxis as well as the treatment of established infections or symptoms.

It will be further appreciated that the amount of a compound of the invention required for use in treatment will vary not only with the particular compound selected but also with the route of administration, the nature of the condition being treated and the age and condition of the patient and will be ultimately at the discretion of the attendant physician or veterinarian. In general however a suitable dose will be in the range from about 1 to about 750 mg/kg of bodyweight per day, such as 3 to about 120 mg per kilogram body weight of the recipient per day, preferably in the range of 6 to 90 mg/kg/day, most preferably in the range of 15 to 60 mg/kg/day.

The desired dose may conveniently be presented in a single dose or as divided doses administered at appropriate intervals, for example as two, three, four or more sub-doses per day.

The compound is conveniently administered in unit dosage form; for example containing lo to 1500 mg, conveniently 20 to 1000 mg, most conveniently 50 to 700 mg of active ingredient per unit dosage form.

Ideally the active ingredient should be administered to achieve peak plasma concentrations of the active compound of from about 1 to 75 µM, preferably about 2 to 50 µM, most preferably about 3 to about 30 µM. This may be achieved, for example, by the intravenous injection of a 0.1 to 5% solution of the active ingredient, optionally in saline, or administered as a bolus containing about 0.1 to about 110 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible that, for use in therapy, a compound of the invention may be administered as the raw chemical it is preferable to present the active ingredient as a pharmaceutical formulation.

The invention thus further provides a pharmaceutical formulation comprising a compound of formula (I) or a pharmaceutically acceptable derivative thereof together with one or more pharmaceutically acceptable carriers therefor and, optionally, other therapeutic and/or prophylactic ingredients. The carrier(s) must be 'acceptable' in the sense of being compatible with the other ingredients of the formulation and not deleterious to the recipient therefor.

Pharmaceutical formulations include those suitable for oral, rectal, nasal, topical (including buccal and sub-lingual), vaginal or parenteral (including intramuscular, sub-cutaneous and intravenous) administration or in a form suitable for administration by inhalation or insufflation. The formulations may, where appropriate, be conveniently presented in discrete dosage units and may be prepared by any of the methods well known in the art of pharmacy. All methods include the step cf bringing into association the active compound with liquid carriers or finely divided solid carriers or both and then, if necessary, shaping the product into the desired formulation.

Pharmaceutical formulations suitable for oral administration may conveniently be presented as discrete units such as capsules, cachets or tablets each containing a predetermined amount of the active ingredient; as a powder or granules; as a solution; as a suspension; or as an emulsion. The active ingredient may also be presented as a bolus, electuary or paste. Tablets and capsules for oral administration may contain conventional excipients such as binding agents, fillers, lubricants, disintegrants, or wetting agents. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils) or preservatives.

The compounds according to the invention may also be formulated for parenteral administration (e.g., by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g., sterile, pyrogen-free water, before use.

For topical administration to the epidermis, the compounds according to the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active ingredient in a flavored based, usually sucrose and acacia or tragacanth; pastilles comprising the active ingredient in an inert base such as gelatin and glycerin or sucrose and acacia; and mouthwashes comprising the active ingredient in a suitable liquid carrier.

Pharmaceutically formulations suitable for rectal administration wherein the carrier is a solid, are most preferably represented as unit dose suppositories. Suitable carriers include cocoa butter and other materials commonly used in the art, and the suppositories may be conveniently formed by admixture of the active compound with the softened or melted carrier(s) followed by chilling and shaping in molds.

Formulations suitable for vaginal administration may be presented as pessaries, tampons, creams, gels, pastes, foams or sprays containing in addition to the active ingredient, such carriers as are known in the art to be appropriate.

For intra-nasal administration the compounds of the invention may be used as a liquid spray or dispersible powder or in the form of drops.

Drops may be formulated with an aqueous or non-aqueous base also comprising one or more dispersing agents, solubilizing agents or suspending agents. Liquid sprays are conveniently delivered from pressurized packs.

For administration by inhalation, the compounds according to the invention are conveniently delivered from an insufflator, nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount.

Alternatively, for administration by inhalation or insufflation, the compounds according to the invention may take the form of a dry powder composition, for example a powder mix of the compound and a suitable powder base such as lactose or starch. The powder composition may be presented in unit dosage form in, for example, capsules or cartridges or e.g., gelatin or blister packs from which the powder may be administered with the aid of an inhalator or insufflator.

When desired, the above described formulations adapted to give sustained release of the active ingredient, may be employed.

The pharmaceutical compositions according to the invention may also contain other active ingredients such as antimicrobial agents, or preservatives.

The compounds of the invention may also be used in combination with other therapeutic agents, for example, other antiinfective agents. In particular the compounds of the invention may be employed together with known antiviral agents.

The invention thus provides, in a further aspect, a combination comprising a compound of formula (I) or a physiologically acceptable derivative thereof together with another therapeutically active agent, in particular, an antiviral agent.

The combinations referred to above may conveniently be presented for use in the form of a pharmaceutical formulation and thus pharmaceutical formulations comprising a combination as defined above together with a pharmaceutically acceptable carrier therefor comprise a further aspect of the invention.

Suitable therapeutic agents for use in such combinations include acyclic nucleosides such as aciclovir, ganciclovir, interferons such as alpha-, beta-and gamma-interferon; glucuronation inhibitors such as probenicid; nucleoside transport inhibitors such as dipyridamole; nucleoside analogues such as 3'-azido-2',3'-dideoxythymidine, 2',3'-dideoxycytidine, 2',3'-dideoxyadenosine, 2',3'-dideoxyinosine, 2',3'-dideoxythymidine, 2',3'-dideoxy-2',3'-didehydrothymidine, and 2',3'-dideoxy-2',3'-didehydrocytidine and ribavirin; immunomodulators such as interleukin II (IL2) and granulocyte macrophage colony stimulating factor (GM-CSF), erythropoietin, ampligen, thymomodulin, thymopentin, foscarnet, glycosylation inhibitors such as 2-deoxy-D-glucose, castanospermine, 1-deoxynojirimycin; and inhibitors of HIV binding to CD4 receptors such as soluble CD4, CD4 fragments and CD4-hybrid molecules.

The individual components of such combinations may be administered either sequentially or simultaneously in separate or combined pharmaceutical formulations.

When the compound of formula (I) or a pharmaceutically acceptable derivative thereof is used in combination with a second therapeutic agent active against the same virus, the dose of each compound may be either the same or differ from that when the compound is used alone. Appropriate doses will be readily appreciated by those skilled in the art.

The compounds of formula (I) and their pharmaceutically acceptable derivatives may be prepared by any method known in the art for the preparation of compounds of analogous structure.

In one such process (A) a 1,3-oxathiolane of formula (VIII) 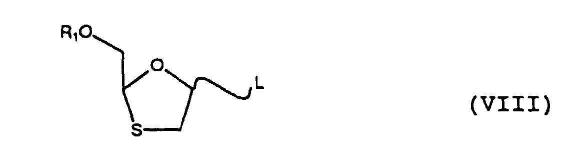 wherein R₁ is hydrogen or hydroxyl protecting group as defined herein and the anomeric group L is a displaceable atom or group, is reacted with an appropriate base. Suitable groups L include alkoxy carbonyl groups such as ethoxy carbonyl or halogens, for example, iodine, bromine or chlorine or -OR where R is a substituted or unsubstituted, saturated or unsaturated alkyl group, e.g., a C₁₋₆ alkyl group such as methyl, or R is a substituted or unsubstituted aliphatic or aromatic acyl group, e.g., a C₁₋₆ aliphatic acyl group such as acetyl and an aromatic acyl group such as benzoyl.

The compound of formula (VIII) is conveniently reacted with the appropriate purine or pyrimidine base R₂-H (previously silylated with a silylating agent such as hexamethyldisilazane) in a compatible solvent such as methylene chloride using a Lewis acid (such as titanium tetrachloride or stannic chloride) or trimethylsilytriflate.

The 1,3-oxathiolanes of formula (VIII) may be prepared, for example, by reaction of an aldehyde of formula (VII) with a mercaptoacetal of formula (VI) in a compatible organic solvent, such an toluene, in the presence of an acid catalyst such an a para-toluene sulfonic acid or a Lewis acid, e.g., zinc chloride.

(VI) HSCH₂CH(OC₂H₅)₂

C₆H₅COOCH₂CHO (VII)

The mercaptoacetals of formula (VI) may be prepared by methods known in the art, for example, G. Hesse and I. Jorder, "Mercaptoacetaldehyde and dioxy-1, 4-dithiane", Chem. Ber 85, pp. 924-932 (1952).

The aldehydes of formula (VII) may be prepared by methods known in the art, for example, E.G. Halloquist and H. Hibbert, "Studies on reactions relating to carbohydrates and polysaccharides. Part XLIV: Synthesis of isomeric bicyclic acetal ethers", Can. J. Research, 8, pp. 129-136 (1933).

In a second process (B) a compound of formula (I') 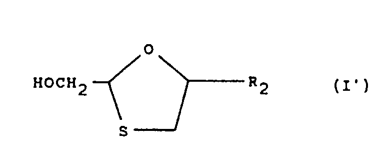 wherein R₂ is a purine or pyrimidine base other than cytosine is converted to a compound of formula (I) by base interconversion. Such interconversion may be effected either by simple chemical transformation (e.g., the conversion of uracil base to cytosine) or by an enzymatic conversion using, for example, a deoxyribosyl transferase. Such methods and conditions for base interconversions are well known in the art of nucleoside chemistry.

In a third process (C) the compounds of formula (I) may be prepared by the reaction of a compound of formula (IX) 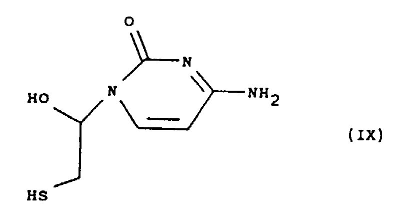 with a compound of formula (X) 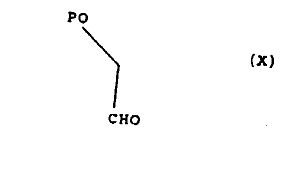 where P is a protecting group, followed by removal of the protecting group.

The compounds of formula (IX) may be prepared for reaction by a suitable epoxide (XI). 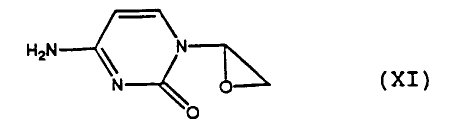 with an appropriate sulphur-containing compound, e.g., sodium thioacetate. Compounds of formula (XI) are either known in the art or may be obtained by analogous processes.

In a fourth process (D) a compound of formula (XII) 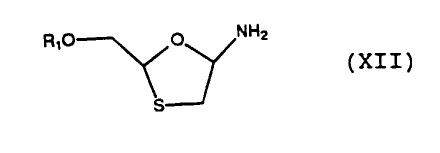 may be converted to a compound of formulae (I) or (II) by conversion of the anomeric NH2 group to the required base by methods well known in the art of nucleoside chemistry.

Many of the reactions described hereinabove have been extensively reported in the context of purine nucleoside synthesis, for example, in "Nucleoside Analogues - Chemistry, Biology and Medical Applications", R.T. Walker et al., Eds, Plenum Press, New York (1979) at pages 193-223, the text of which is incorporated by reference herein.

It will be appreciated that the above reactions may require the use of, or conveniently may be applied to, starting materials having protected functional groups, and deprotection might thus be required as an intermediate or final step to yield the desired compound. Protection and deprotection of functional groups may be effected using conventional means. Thus, for example, amino groups may be protected by a group selected from aralkyl (e.g., benzyl), acyl or aryl (e.g., 2,4-dinitrophenyl); subsequent removal of the protecting group being effected when desired by hydrolysis or hydrogenolysis as appropriate using standard conditions. Hydroxyl groups may be protected using any conventional hydroxyl protecting group, for example, as described in "Protective Groups in Organic Chemistry", Ed. J.F.W. McOmie (Plenum Press, 1973) or "Protective Groups in Organic synthesis" by Theodora W. Greene (John Wiley and Sons, 1981). Examples of suitable hydroxyl protecting groups include groups selected from alkyl (e.g., methyl, t-butyl or methoxymethyl), aralkyl (e.g., benzyl, diphenylmethyl or triphenylmethyl), heterocyclic groups such as tetrahydropyranyl, acyl, (e.g., acetyl or benzoyl) and silyl groups such as trialkylsilyl (e.g., t-butyldimethylsilyl). The hydroxyl protecting groups may be removed by conventional techniques. Thus, for example, alkyl, silyl, acyl and heterocyclic groups may be removed by solvolysis, e.g., by hydrolysis under acidic or basic conditions. Aralkyl groups such as triphenylmethyl may similarly be removed by solvolysis, e.g., by hydrolysis under acidic conditions. Aralkyl groups such as benzyl may be cleaved, for example, by treatment with Bf₃/etherate and acetic anhydride followed by removal of acetate groups so formed at an appropriate stage in the synthesis. Silyl groups may also conveniently be removed using a source of fluoride ions such as tetra-n-butylammonium fluoride.

In the above processes the compounds of formula (I) are generally obtained as a mixture of the cis and trans isomers.

These isomers may be separated, for example, by acetylation, e.g., with acetic anhydride followed by separation by physical means, e.g., chromatography on silica gel and deacetylation, e.g., with methanolic ammonia or by fractional crystallization.

Pharmaceutically acceptable salts of the compounds of the invention may be prepared as described in United States Patent No. 4,383,114, the disclosure of which is incorporated by reference herein. Thus, for example, when it is desired to prepare an acid addition salt of a compound of formula (I), the product of any of the above procedures may be converted into a salt by treatment of the resulting free base with a suitable acid using conventional methods. Pharmaceutically acceptable acid addition salts may be prepared by reacting the free base with an appropriate acid optionally in the presence of a suitable solvent such as an ester (e.g., ethyl acetate) or an alcohol (e.g., methanol, ethanol or isopropanol). Inorganic basic salts may be prepared by reacting the free base with a suitable base such as an alkoxide (e.g., sodium methoxide) optionally in the presence of a solvent such as an alcohol (e.g., methanol). Pharmaceutically acceptable salts may also be prepared from other salts, including other pharmaceutically acceptable salts, of the compounds of formula (I) using conventional methods.

A compound of formula (I) may be converted into a pharmaceutically acceptable phosphate or other ester by reaction-with a phosphorylating agent, such as Pocl₃, or a suitable esterifying agent, such as an acid halide or anhydride, as appropriate. An ester or salt of a compound of formula (I) may be converted to the parent compound, for example, by hydrolysis.

Where the compound of formula (I) is desired as a single isomer it may be obtained either by resolution of the final product or by stereospecific synthesis from isomerically pure starting material or any convenient intermediate.

Resolution of the final product, or an intermediate or starting material therefore may be effected by any suitable method known in the art: see for example, stereochemistry of Carbon Compounds, by E.L. Eliel (McGraw Hill, 1962) and Tables of Resolvina Agents, by S.H. Wilen.

The invention will be further described by the following examples which are not intended to limit the invention in any way. All temperatures are in degrees celsius.

### EXAMPLES

### Example 1

### 2-(benzoylthio) acetaldehyde diethylacetal

C₆H₅COS-CH₂CH(OC₂H₅)₂ (V)

To a solution of potassium t-butoxide (11.5 g. 0.11 mol) in DMF (100 ml) was added thiobenzoic acid (17 g. 0.11 mol) and the solution partially evaporated in vacuo, benzene added in two consecutive portions (2 x 30 ml) and evaporated in vacuo each time. To the residual DMF solution was added bromoacetaldehyde diethylacetal (20.3 g. 0.1 mol) and the mixture stirred at 120° for 15 h. After cooling, it was poured onto water (500 ml), the product extracted with ether (3 x 200 ml), the extract washed with aqueous NaHCO₃ followed by water, then dried and the solvent removed in vacuo. The residue was distilled in vacuo to give 17.2 g. of pure (V), b.p. 131-133°/0.07 mm. It was characterized by ¹H NMR δ(ppm in CDCl₃):
7.97 (d, 2H; aromatic)
7.47 (m, 3H; aromatic)
4.59 (t, 1H; -CH(OC₂H₅)₂))
3.66 (m, 4H; 2 x OCH₂CH₃)
3.30 (d, 2H; SCH₂-)
1.23 (t, 6H; 2 x OCH₂CH₃)

### Example 2

### Mercaptoacetaldehyde diethylacetal

HSCH₂CH(OC₂H₅)₂ (VI)

The preceding benzoylthio derivative (V) (17.2 g) was dissolved in 100 ml THF followed by the addition of 6 g NaOH in 20 ml H₂O. THF mixture was with refluxed under N₂ for 15 h, then cooled and diluted with water (200 ml) and the product extracted with ether (3 x 200 ml). The extract was dried, the solvent removed in vacuo and the residue distilled in vacuo to yield 7.1 g of pure (VI), b.p. 60-62°/18 mm. It was characterized by ¹H NMR δ(ppm in CDCl₃):
4.51 (t, 1H; CH(OC₂H₅)₂)
3.51 (m, 4H; 2 x OCH₂CH₃)
2.65 (dd, 2H; HS-CH₂) .
1.54 (t, 1H; HS-)
1.23 (t, 6H; 2 x OCH₂CH₃)

### Example 3

### Benzoyloxyacetaldehyde

C₆H₅COOCH₂CHO (VII)

This known intermediate was prepared by a previously unreported method from the known 1-benzoyl glycerol. Thus, 50 g of the latter in a mixture of 500 ml of CH₂Cl₂ and 25 ml of H₂O was treated portionwise with 80 g of NaIO under vigorous stirring at room temperature. After addition, stirring was continued for 2 h after which time 100 g of MgSO₄ was added and stirring continued for 30 min. The mixture was filtered, the filtrate evaporated in vacuo and the residue distilled in vacuo to yield 26 g of pure (VII) b.p. 92-94°/0.25 mm. ¹H NMR (200 MH_{z}; TMS as internal reference)
δ(ppm in CDCl₃,):
9.71 (s, 1H; -CHO)
8.11 (d, 2H; aromatic)
7.60 (m, 1H; aromatic)
7.46 (m, 2H; aromatic)
4.88 (s, 2H; -CH₂CHO)

### Example 4

### 2-Benzoyloxymethyl-5-ethoxy-1,3-oxathiolane

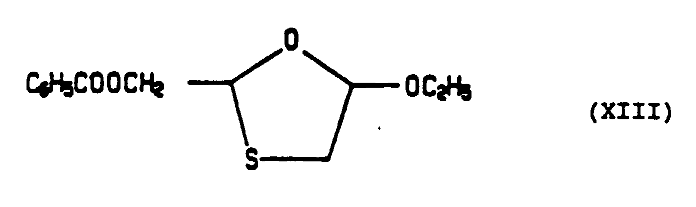

The preceding mercaptoacetaldehyde acetal (VI) (7 g) was mixed in 100 ml of toluene with 7 g of the above benzoyloxyacetaldehyde (VII), a few crystals of para-toluene sulfonic acid added and the mixture placed in an oil-bath at 120° under N₂. The formed ethanol was allowed to distill over, the mixture kept at 120° for an additional 30 minutes, then cooled and washed with aqueous NaHCO₃, dried and evaporated in vacuo. The residue was distilled in vacuo to yield 9.8 g of pure (XIII) as a mixture of cis- and trans-isomers, b.p. 140-143°/0.1 mm; R_{f} 0.51 (hexane-EtOAc);
¹H NMR δ(ppm in CDCl₃):
8.05 (m, 2H; aromatic)
7.57 (m, 1H; aromatic)
7.43 (m, 2H; aromatic)
5.55 (m, 2H; C₅-H, C₂-H)
4.55 (m, 2H; C₂-C₆H₅-CO₂CH₂)
3.80 (m, 1H ; C₂-C₆H₅CO₂CH₂)
3.76 (m, 1H; 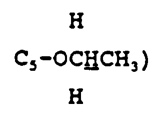
3.17 (m, 2H; C₄-H₂)
1.21 (t, 3H; C₅-OCH₂CH₃)

### Example 5

### Cis- and trans-2-benzoyloxymethyl-5-(cytosin-1'-yl)-1,3-oxathiolanes

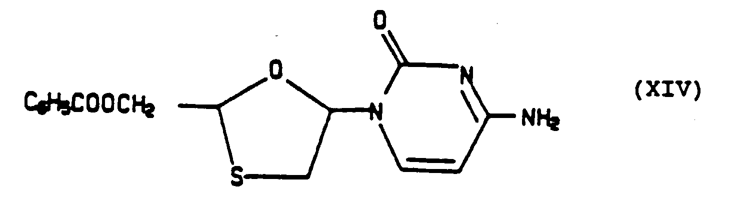

A mixture of 2.7 g of cytosine, 30 ml of hexamethyldisilazane (HMDS) and 0.3 ml of trimethylsilyl chloride (TMSCl) was heated under reflux under dry N₂ until a clear solution resulted (3 hours) and the excess reagents evaporated in vacuo. The remaining volatiles were removed under high vacuum (15 min.), the solid residue taken up in 250 ml of 1, 2-dichloroethane and 5 g of the above key intermediate (XIII) in 50 ml of dichloroethane added under dry argon followed by 4.7 ml of trimethylsilyl triflate (TMST_{f}). After 3 days of heating under reflux under argon, it was cooled and poured onto 300 ml of saturated aqueous NaHCO₃. The organic layer was collected, the aqueous phase extracted with CH₂Cl₂ (2 X 100 ml) and the combined extracts washed with water, dried and evaporated in vacuo. The residue was purified by chromatography on silica gel using CH₂Cl₂:CH₃OH 9:1 as the eluant to give 2.5 g of a pure mixture of cis- and trans-(XIV) in a 1:1 ratio as ascertained by ¹H NMR. These were separated as the N-acetyl derivatives as described in the following example.

### Example 6

### Cis- and trans-isomers of 2-benzoyloxymethyl-5-(N₄'-acetyl-cytosin-1'-yl)-1.3-oxathiolane

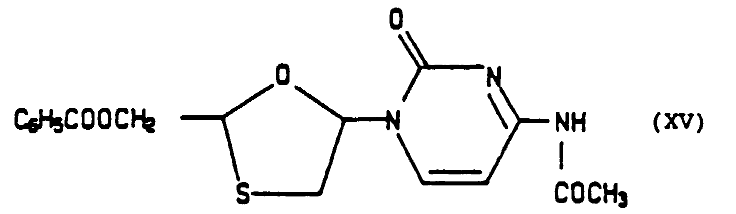

The preceding mixture (XIV) (2.5 g) in l00 ml of dry pyridine containing 0.1 g of 4-dimethylaminopyridine (DMAP) was treated with acetic anhydride (7 ml) at room temperature and after 16 hours, the mixture was poured onto cold water followed by extraction with CH₂Cl₂ (3 X 150 ml). The extract was washed with water, dried, and evaporated in vacuo. Toluene was added to the residue, then evaporated in vacuo and the residual oil purified by chromatography on silica gel using EtOAc:CH₃OH 99:1 as the eluant to yield 1.35 g of pure trans-(XV) as the fast moving product and 1.20 g of pure cis-(XV) as the slow moving component. These were characterized by ¹H NMR spectroscopy.
trans-(XV): m.p. 158-160°; R_{f}: 0.48 EtOAc:CH₃OH 95:5
U.V.: (CH₃OH) Lambda max: 297 nm
¹H NMR δ(ppm in CDCl₃):
9.00 (b, 1N; C₄'-NH-Ac)
8.06 (m, 2H; aromatic)
7.74 (d, 1H; C₆'-H)
7.56 (m, 1H; aromatic)
7.47 (d, 1H; C₅'-H)
7.45 (m, 2H; aromatic)
6.53 (dd, 1H; C₅-H)
5.89 (dd, 1H; C₂-H)
4.46 (dd, 2H; C₂-CH₂OCOC₆H₅)
3.66 (dd, 1H; C₄-H)
3.32 (dd, 1H; C₄-H)
2.25 (s, 3H; NH-COCH₃)
Cis-(XV): m.p. 150-152°; R_{f}: 0.40 EtOAc:MeOH 95:5)
U.V.: (CH₃OH) Lambda max: 297 nm
¹H NMR δ (ppm in CDCl₃):
9.03 (b, 1H; NH-Ac)
8.21 (d, 1H; C₆'-H)
8.05 (m, 2H; aromatic)
7.60 (m, 1H; aromatic)
7.50 (m, 2H; aromatic)
7.29 (d, 1H; C₅'-H)
6.34 (dd, 1H; C₅-H)
5.52 (dd, 1H; C₂-H)
4.80 (dd, 2H; C₂-CH₂OCOC₆H₅)
3.66 (dd, 1H; C₄-H)
3.24 (dd, 1H; C₄-H)
2.23 (s, 3H; NH-COCH₃)

### Example 7

### Cis- and trans-2-hydroxymethyl-5-(cytosin-1'-yl)-1,3-oxathiolanes

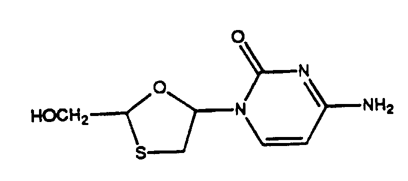

a) Trans-(III): 375 mg of the preceding trans-(XV) was dissolved in 100 ml of methanolic ammonia at 24° and after stirring for 16 hours, the solvent was removed in vacuo and the residue crystallized with ether. It was recrystallized from ethanol-ether to yield 174 mg of pure product, m.p. >220° (dec). It was characterized by ¹H and ¹³C NMR.
¹H NMR δ (ppm in DMSO-d₆) :
7.57 (d, 1H; C₆'-H)
7.18 (d, 2H; C₄'-NH₂)
6.30 (dd, 1H, C₅-H)
5.68 (d, 1H; C₅'-H)
5.48 (t, 1H; C₂-H)
5.18 (t, 1H; C₂-CH₂OH)
3.45 (m, 3H; C₂-CH₂OH + C₄H)
3.06 (dd, 1H; C₄-H)
U.V.: (CH₃OH) Lambda max: 270 nm
¹³C NMR (DMSO-d₆, Varian XL-300); δ in ppm:

| C₂ ^{'} | C₄ ^{'} | C₅ ^{'} | C₆ ^{'} | C₅ | C₄ | C₂ | CH₂OH |
|---|---|---|---|---|---|---|---|
| 154.71 | 165.70 | 93.47 | 140.95 | 87.77 | 36.14 | 86.80 | 64.71 |

b) Cis-(II): treating 375 mg of cis-(XV) by the same preceding procedure led to 165 mg of pure product after recrystallization from ethanol-ether, m.p. 171-173°. It was characterized by ¹H and ¹³C NMR.
¹H NMR: δ (ppm in DMSO-d₆):
7.80 (d, 1H; C₆'-H)
7.20 (d, 2H; C₄'-NH₂)
6.18 (t, 1H; C₅-H)
5.70 (d, lH; C₅'-H)
5.14 (t, lH; C₂-CH₂OH)
3.71 (m, 2H; C₂-CH₂OH)
3.40 (dd, 1H; C₄-H)
2.99 (dd, 1H; C₄-H)
U.V.: (CH₃OH) Lambda max: 270 nm
¹³C NMR δ (ppm in DMSO-d₆)

| C₂ ^{'} | C₄ ^{'} | C₅ ^{'} | C₆ ^{'} | C₅ | C₄ | C₂ | CH₂OH |
|---|---|---|---|---|---|---|---|
| 154.63 | 165.59 | 93.86 | 140.91 | 86.47 | 36.22 | 85.75 | 62.79 |

### Example 8

### Tablet Formulations

A. The following formulation is prepared by wet granulation of the ingredients with a solution of povidone in water, drying and screening, followed by addition of magnesium stearate and compression.

| | mg/tablet |
|---|---|
| (a) Active ingredient | 250 |
| (b) Lactose B.P. | 210 |
| (c) Povidone B.P. | 15 |
| (d) Sodium Starch Glycolate | 20 |
| (e) Magnesium Stearate | 5 |
| | $\overline{\text{500}}$ |

B. The following formulation is prepared by direct compression; the lactose is of the direct compression type.

| | mg/tablet |
|---|---|
| Active ingredient | 250 |
| Lactose | 145 |
| Avicel | 100 |
| Magnesium Stearate | |
| | $\overline{\text{500}}$ |

C. (Controlled Release Formulation) The formulation is prepared by wet granulation of the ingredients (below) with a solution of povidone in water, drying and screening followed by the addition of magnesium stearate and compression.

| | mg/tablet |
|---|---|
| (a) Active ingredient | 500 |
| (b) Hydroxypropylemethylcellulose (Methocel K4M Premium) | 112 |
| (c) Lactose B.P. | 53 |
| (d) Povidone B.P. | 28 |
| (e) Magnesium Stearate | 7 |
| | $\overline{\text{700}}$ |
| | |

### Example 9

### Capsule Formulation

A capsule formulation is prepared by admixing the ingredients below and filling into a two-part hard gelatin capsule.

| | mg/capsule |
|---|---|
| Active ingredient | 125 |
| Lactose | 72.5 |
| Avicel | 50 |
| Magnesium Stearate | 2.5 |
| | $\overline{\text{250}}$ |

### Example 10

### Injectable Formulation

Active ingredient 0.200 g
Sodium hydroxide solution, 0.1M q.s. to a pH of about 11.
Sterile water q.s. to 10 ml.

The active ingredient is suspended in some of the water (which may be warmed) and the pH adjusted to about 11 with a solution of sodium hydroxide. The batch is then made up to volume and filtered through a sterilizing grade membrane filter into a sterile 10 ml glass vial and sealed with sterile closures and overseas.

### Example 11

### suppository

| | mg/suppository |
|---|---|
| Active ingredient | 250 |
| Hard Fat, B.P. | 1770 |
| | 2020 |

One-fifth of the hard fat is melted in a steam-jacketed pan at 45°C maximum. The active ingredient is sifted through a 200 µm sieve and added to the molten base with mixing, using a high shear stirrer, until a smooth dispersion is achieved. Maintaining the mixture at 45°C, the remaining hard fat is added to the suspension and stirred to ensure a homogenous mix. The entire suspension is passed through a 250 µm stainless steel screen and, with continuous stirring, is allowed to cool to 40°C. At a temperature of 38°C to 40°C, 2.02 g of the mixture is filled into suitable, 2 ml plastic molds. The suppositories are allowed to cool to room temperature.

### Example 12

### Antiviral Activity

In vitro testing was conducted on the compound of this invention and AZT to determine their inhibitory properties. The results are shown in Tables 1 and 2. The concentrations reported are µg/ml in the incubation media which affect the susceptibility of a continuous line of T-cells developed at the Lady Davis Institute for Medical Research (Montreal) by Dr. Mark A. Wainberg toward infection by HIV-1 following a protocol similar to that of H. Mitsuya and S. Broder, "Inhibition of the in vitro infectivity and cytopathic effect of human T-lymphotropic virus type III/lymphadenopathy-associated virus (HTLV-III/LAV) by 2'3'-dideoxy-nucleosides", Proc. Natl. Acad. Sci, USA, 83, pp. 1911-15 (1986). Protection of the cell line from infection was monitored by staining with monoclonal antibodies against viral proteins in the standard manner (Table 1). In all experiments, comparisons were made with the drug AZT as the control. In order to confirm the results, the drug effects were monitored by measuring reverse transcriptase (RT) activity in the U-937 line of human monocytic cells as assayed in the usual manner with tritiated thymidine triphosphate (TTP) (Table 2). Finally, the drug effects on cell viability as measured by the well-known cytolytic effects of HIV-1 on the MT-4 cell line was evaluated in the accepted manner (Table 1).

### Toxicity

No toxic effects were observed in the above tests.

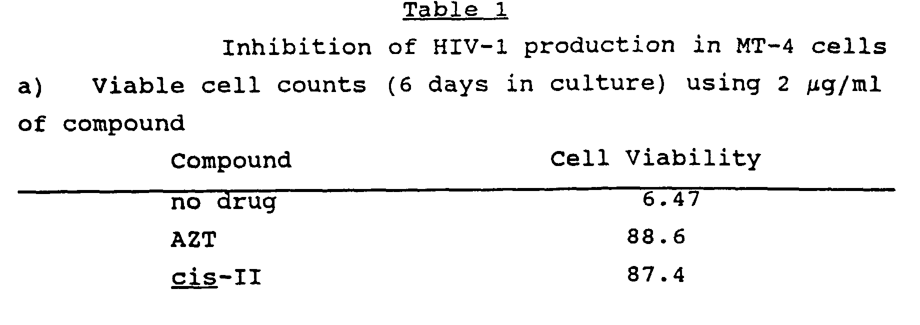

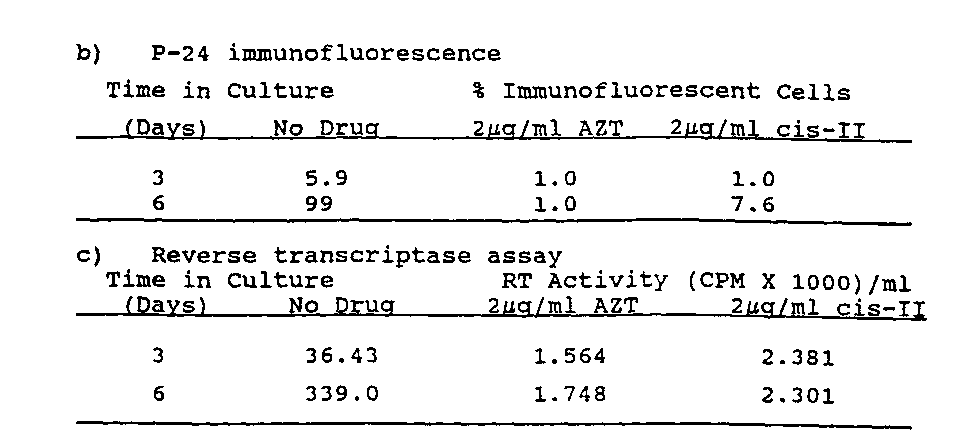

**Table 2**

| Inhibition of HIV-1 production in H-9 cells Reverse transcriptase assay | | | |
|---|---|---|---|
| Time in Culture | | RT Activity (CPM X 1000)/ml | |
| (Days) | No Drug | 2µ/ml AZT | 2µg/ml cis-II |
| 5 | 9.117 | 3.346 | 3.077 |
| 8 | 438.5 | 3.414 | 5.853 |
| 11 | 2550 | 2.918 | 3.560 |
| 14 | 2002 | 8.320 | 2.872 |
| 17 | 584.5 | 2.997 | 2.399 |
| 21 | 365.2 | 3.111 | 2.907 |
| 25 | 436.4 | 15.88 | 4.020 |
| 29 | 92.38 | 32.08 | 3.756 |
| 33 | 111.1 | 612.2 | 3.803 |
| 37 | 32.28 | 878.2 | 4.193 |
| 41 | 384.4 | 994.0 | 4.515 |
| 45 | 33.64 | 32.91 | 3.441 |

## Claims (Claims for the following Contracting State(s): AT, BE, CH, DE, DK, FR, GB, IT, LI, LU, NL, SE)

1. A cis-1.3-oxathiolane of formula 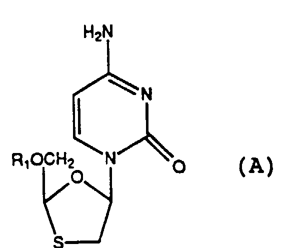 wherein R₁ represents hydrogen, a C₁₋₁₆ alkanoyl group, a benzoyl group or a benzoyl group substituted by at least one halogen, C₁₋₆ alkyl, C₁₋₆ alkoxy, nitro or trifluoromethyl group, or a pharmaceutically acceptable salt thereof in the form of a single optical isomer or a mixture of optical isomers.

2. A compound according to claim 1 wherein the compound is cis-2-hydroxymethyl-5-(cytosin-1'-yl)-1,3-oxathiolane or a pharmaceutically acceptable salt thereof.

3. A compound according to Claim 2 in the form of a racemic mixture.

4. A compound according to Claim 2 substantially in the form of a single enantiomer.

5. A compound according to any of Claims 2 to 4 wherein the compound is cis-2-hydroxymethyl-5-(cytosin-1'-yl)-1,3-oxathiolane.

6. A compound according to any preceding claim for use in the manufacture of a medicament for the treatment of a viral infection.

7. A pharmaceutical formulation comprising a compound according to any of Claims 2 to 5 together with a pharmaceutically acceptable carrier therefor.

8. A pharmaceutical formulation according to Claim 7 additionally comprising a further therapeutic agent.

9. A process for the preparation of a compound as claimed in any of Claims 2 to 5 which comprises:
(a) reaction of a compound of formula (VIII) 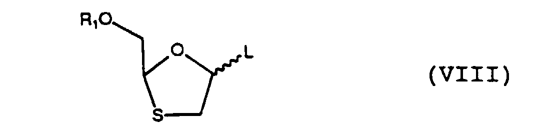 wherein R₁ is hydrogen or a hydroxyl protecting group and L is a displaceable atom or group with cytosine;
(b) base interconversion of compound of formula (I') 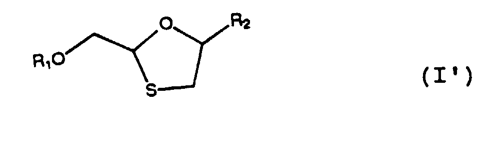 wherein R₂ is a purine or pyrimidine base other than cytosine into cis-2-hydroxymethyl-5-(cytosin-1'-yl)-1,3-oxathiolane;
(c) reaction of a compound of formula (IX) 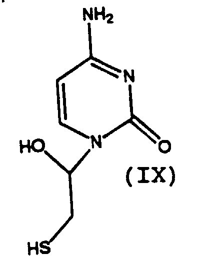 with a compound of formula (X) 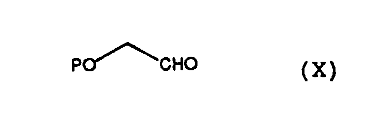 wherein P is a protecting group; or
(d) conversion of a compound of formula (XII) 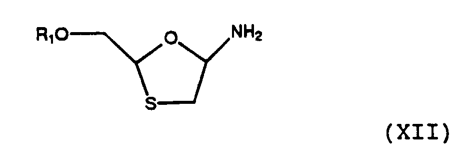 to cis-2-hydroxymethyl-5-(cytosin-1'-yl)-1,3-oxathiolane
and if necessary or desired subjecting the compound resulting from any of steps (a) to (d) to one or two further reactions comprising:
(i) removing any protecting groups;
(ii) converting cis-2-hydroxymethyl-5-(cytosin-1'-yl)-1,3-oxathiolane or a salt thereof into a pharmaceutically acceptable salt thereof.

10. A process according to Claim 9 wherein the obtained compound is in the form of a racemic mixture.

11. A process according to Claim 9 wherein the obtained compound is substantially in the form of a single enantiomer.

12. A process according to any one of Claims 9 to 11 wherein in step (a) the group L is selected from a group consisting of alkoxy carbonyl, iodine, bromine, chlorine or -OR, where R is a substituted or unsubstituted, saturated or unsaturated alkyl group or R is a substituted or unsubstituted aliphatic or aromatic acyl group.

13. A process according to any one of Claims 9 to 12 wherein in step (a) the compound of formula (VIII) is reacted with a silylated cytosine in a compatible solvent in the presence of a Lewis acid or trimethylsilyltriflate.

14. A method for the preparation of a pharmaceutical formulation comprising admixing a compound as claimed in any of Claims 2 to 5 with a pharmaceutically acceptable carrier therefor.

## Claims (Claims for the following Contracting State(s): ES, GR)

1. A process for the preparation of a cis-1,3-oxathiolane of formula 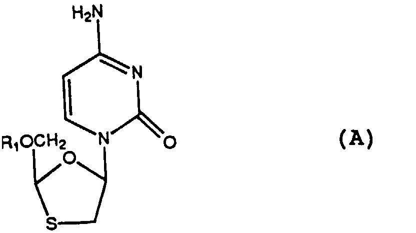 wherein R₁ represents hydrogen, a C₁₋₁₆ alkanoyl group, a benzoyl group or a benzoyl group substituted by at least one halogen, C₁₋₆ alkyl, C₁₋₆ alkoxy, nitro or trifluoromethyl group, or a pharmaceutically acceptable salt thereof in the form of a single optical isomer or a mixture of optical isomers which comprises:
(a) reaction of a compound of formula (VIII) 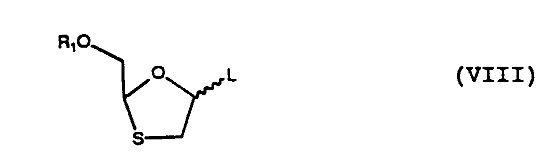 wherein R₁ is hydrogen or a hydroxyl protecting group and L is a displaceable atom or group with cytosine;
(b) base interconversion of compound of formula (I') 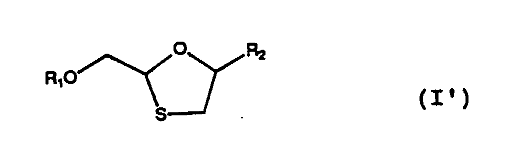 wherein R₂ is a purine or pyrimidine base other than cytosine into cis-2-hydroxymethyl-5-(cytosin-1'-yl)-1,3-oxathiolane;
(c) reaction of a compound of formula (IX) 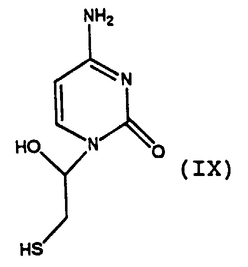 with a compound of formula (X) 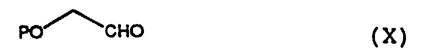 wherein P is a protecting group; or
(d) conversion of a compound of formula (XII) 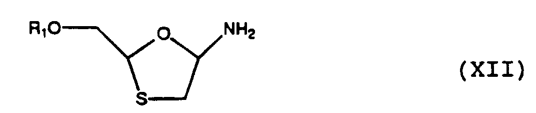 to cis-2-hydroxymethyl-5-(cytosin-1'-yl)-1,3-oxathiolane
and if necessary or desired subjecting the compound resulting from any of steps (a) to (d) to one or two further reactions comprising:
(i) removing any protecting groups;
(ii) converting cis-2-hydroxymethyl-5-(cytosin-1'-yl)-l,3-oxathiolane or a salt thereof into a pharmaceutically acceptable salt thereof.

2. A process according to Claim 1 wherein the obtained compound is cis-2-hydroxymethyl-5-(cytosin-1'-yl)-1,3-oxathiolane or a pharmaceutically acceptable salt thereof.

3. A process according to Claim 2 wherein the obtained compound is in the form of a racemic mixture.

4. A process according to Claim 2 wherein the obtained compound is substantially in the form of a single enantiomer.

5. A process according to any preceding claim wherein the obtained compound is cis-2-hydroxymethyl-5-(cytosin-1'-yl)-1,3-oxathiolane.

6. A process according to any one of Claims 2 to 5 wherein in step (a) the group L is selected from a group consisting of alkoxy carbonyl, iodine, bromine, chlorine or -OR, where R is a substituted or unsubstituted, saturated or unsaturated alkyl group or R is a substituted or unsubstituted aliphatic or aromatic acyl group.

7. A process according to any one of Claims 2 to 6 wherein in step (a) the compound of formula (VIII) is reacted with a silylated cytosine in a compatible solvent in the presence of a Lewis acid or trimethylsilyltriflate.

## Patentansprüche (Patentansprüche für folgende(n) Vertragsstaat(en): AT, BE, CH, LI, DE, DK, FR, GB, IT, LU, NL, SE)

1. cis-1,3-oxathiolan der Formel 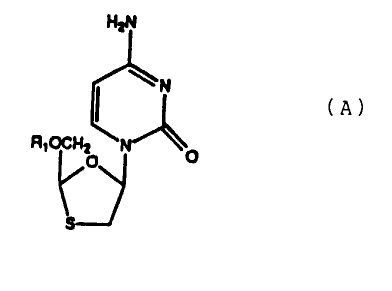 in der R₁ Wasserstoff, einen C₁₋₁₆-Alkanoylrest, einen Benzoylrest oder einen mindestens jeweils durch Halogen, C₁₋₆-Alkyl, C₁₋₆-Alkoxy, Nitro oder Trifluormethyl substituierten Benzoylrest darstellt,
oder ein pharmazeutisch geeignetes Salz desselben in Form eines einzelnen optischen Isomeren oder in Form eines Gemisches der optischen Isomeren.

2. Verbindung gemäß Anspruch 1, wobei die Verbindung ein cis-2-Hydroxymethyl-5-(cytosin-1'-yl)-1,3-oxathiolan oder ein pharmazeutisch geeignetes Salz davon ist.

3. Verbindung gemäß Anspruch 2 in Form eines Racematgemisches.

4. Verbindung gemäß Anspruch 2 im wesentlichen in Form eines einzelnen Enantiomeren.

5. Verbindung gemäß einem der Ansprüche 2 bis 4, bei der es sich um cis-2-Hydroxymethyl-5-(cytosin-1'-yl)-1,3-oxathiolan handelt.

6. Verbindung gemäß einem der vorstehenden Ansprüche zur Verwendung bei der Herstellung eines Arzneimittels zur Behandlung von Virusinfektionen.

7. Pharmazeutische Zubereitung enthaltend eine Verbindung gemäß einem der Ansprüche 2 bis 5 zusammen mit einem dafür geeigneten pharmazeutischen Träger.

8. Pharmazeutische Zubereitung gemäß Anspruch 7, welche zusätzlich einen weiteren therapeutischen Wirkstoff enthält.

9. Verfahren zur Herstellung der Verbindungen gemäß einem der Ansprüche 2 bis 5, umfassend:
(a) die Umsetzung einer Verbindung der Formel (VIII) 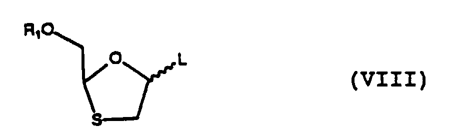 in der R₁ Wasserstoff oder eine Hydroxylschutzgruppe darstellt und L ein durch Cytosin austauschbares Atom oder eine austauschbare Gruppe ist,
(b) die Basenumwandlung der Verbindung der Formel (I') 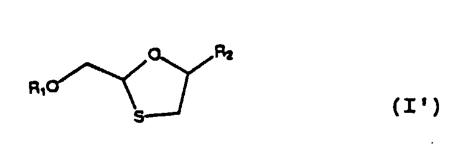 in der R₂ eine andere Purin- oder Pyrimidinbase als Cytosin ist, zu cis-2-Hydroxymethyl-5-cytosin-1'-yl)-1,3-oxathiolan,
(c) die Umsetzung einer Verbindung der Formel (IX) 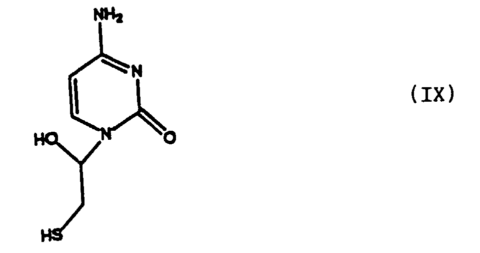 mit einer Verbindung der Formel (X) 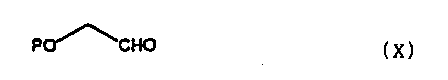 worin P eine Schutzgruppe darstellt, oder
(d) die Umwandlung einer Verbindung der Formel (XII) 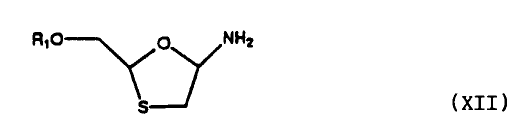 zu cis-2-Hydroxymethyl-5-(cytosin-1'-yl)-1,3-oxathiolan,
und, falls notwendig oder gewünscht, Unterwerfen der Verbindung, die aus einem der Schritte (a) bis (d) erhalten wird, unter eine oder zwei weitere Reaktionen von:
(i) Entfernen aller Schutzgruppen,
(ii) Umwandlung von cis-2-Hydroxymethyl-5-(cytosin-1'-yl)-1,3-oxathiolan oder eines Salzes davon in ein pharmazeutisch geeignetes Salz desselben.

10. Verfahren gemäß Anspruch 9, bei dem die erhaltene Verbindung in Form eines Racematgemisches vorliegt.

11. Verfahren gemäß Anspruch 9, bei dem die Verbindung im wesentlichen in Form eines einzelnen Enantiomeren vorliegt.

12. Verfahren gemäß einem der Ansprüche 9 bis 11, bei dem in Schritt (a) die Gruppe L aus einer aus Alkoxycarbonyl, Jod, Brom, Chlor oder -OR bestehenden Gruppe ausgewählt ist, wobei R ein substituierter oder unsubstituierter, gesättigter oder ungesättigter Alkylrest oder ein substituierter oder unsubstituierter aliphatischer oder aromatischer Acylrest ist.

13. Verfahren gemäß einem der Ansprüche 9 bis 12, bei dem in Schritt (a) die Verbindung der Formel (VIII) mit einem silylierten Cytosin in einem passenden Lösungsmittel in Anwesenheit einer Lewissäure oder von Trimethylsilyltriflat umgesetzt wird.

14. Verfahren zur Herstellung einer pharmazeutischen Zubereitung, umfassend das Mischen einer Verbindung gemäß einem der Ansprüche 2 bis 5 mit einem dafür geeigneten pharmazeutischen Träger.

## Patentansprüche (Patentansprüche für folgende(n) Vertragsstaat(en): ES, GR)

1. Verfahren zur Herstellung eines cis-1,3-Oxathiolans der Formel 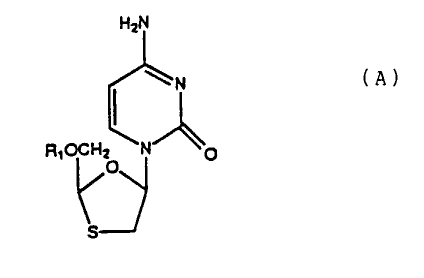 in der R₁ Wasserstoff, einen C₁₋₁₆-Alkanoylrest, einen Benzoylrest oder einen mindestens jeweils durch Halogen, C₁₋₆-Alkyl, C₁₋₆-Alkoxy, Nitro oder Trifluormethyl substituierten Benzoylrest darstellt,
oder ein pharmazeutisch geeignetes Salz desselben in Form eines einzelnen optischen Isomeren oder in Form eines Gemisches der optischen Isomeren,
umfassend:
(a) die Umsetzung einer Verbindung der Formel (VIII) 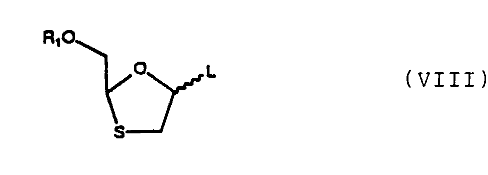 in der R₁ Wasserstoff oder eine Hydroxylschutzgruppe darstellt und L ein durch Cytosin austauschbares Atom oder eine austauschbare Gruppe ist,
(b) die Basenumwandlung der Verbindung der Formel (I') 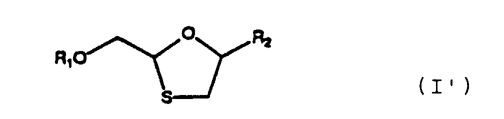 in der R₂ eine andere Purin- oder Pyrimidinbase als Cytosin ist, zu cis-2-Hydroxymethyl-5-(cytosin-1'-yl)-1,3-oxathiolan,
(c) die Umsetzung einer Verbindung der Formel (IX) 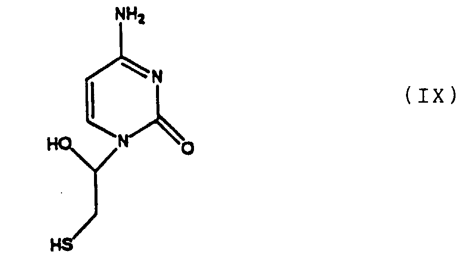 mit einer Verbindung der Formel (X) 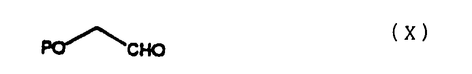 worin P eine Schutzgruppe darstellt, oder
(d) die Umwandlung einer Verbindung der Formel (XII) 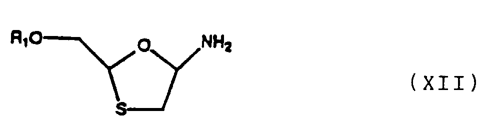 zu cis-2-Hydroxymethyl-5-(cytosin-1'-yl)-1,3-oxathio-lan,
und, falls notwendig oder gewünscht, Unterwerfen der Verbindung, die aus einem der Schritte (a) bis (d) erhalten wird, unter eine oder zwei weitere Reaktionen von:
(i) Entfernen aller Schutzgruppen,
(ii) Umwandlung von cis-2-Hydroxymethyl-5-(cytosin-1'-yl)-1,3-oxathiolan oder eines Salzes davon in ein pharmazeutisch geeignetes Salz desselben.

2. Verfahren gemäß Anspruch 1, bei dem die erhaltene Verbindung cis-2-Hydroxymethyl-5-(cytosin-1'-yl)-1,3-oxathiolan oder ein pharmazeutisch geeignetes Salz desselben darstellt.

3. Verfahren gemäß Anspruch 2, bei dem die erhaltene Verbindung in Form eines Racematgemisches vorliegt.

4. Verfahren gemäß Anspruch 2, bei dem die Verbindung im wesentlichen in Form eines einzelnen Enantiomeren vorliegt.

5. Verfahren gemäß einem der vorstehenden Ansprüche, bei dem die erhaltene Verbindung cis-2-Hydroxymethyl-5-(cytosin-1'-yl)-1,3-oxathiolan ist.

6. Verfahren gemäß einem der Ansprüche 2 bis 5, bei dem in Schritt (a) die Gruppe L aus einer aus Alkoxycarbonyl, Jod, Brom, Chlor oder -OR bestehenden Gruppe ausgewählt ist, wobei R ein substituierter oder unsubstituierter, gesättigter oder ungesättigter Alkylrest oder ein substituierter oder unsubstituierter aliphatischer oder aromatischer Acylrest ist.

7. Verfahren gemäß einem der Ansprüche 2 bis 5, bei dem in Schritt (a) die Verbindung der Formel (VIII) mit einem silylierten Cytosin in einem passenden Lösungsmittel in Anwesenheit einer Lewissäure oder von Trimethylsilyltriflat umgesetzt wird.

## Revendications (Revendications pour l'(les) Etat(s) contractant(s) suivant(s): AT, BE, CH, DE, DK, FR, GB, IT, LI, LU, NL, SE)

1. Cis-1,3-oxathiolane de formule : 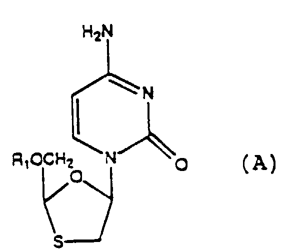 dans laquelle R₁ représente un atome d'hydrogène, un groupe alcanoyle C₁₋₁₆, un groupe benzoyle, ou un groupe benzoyle substitué par au moins un halogène, un groupe alkyle C₁₋₆, alcoxy C₁₋₆, nitro, ou trifluorométhyle, ou sel de celui-ci, pharmaceutiquement acceptable, sous la forme d'un isomère optique unique, ou d'un mélange d'isomères optiques.

2. Composé selon la revendication 1, dans lequel le composé est le cis-2-hydroxyméthyl-5-(cytosin-1'-yl)-1,3-oxathiolane, ou un sel de celui-ci, pharmaceutiquement acceptable.

3. Composé selon la revendication 2, sous la forme d'un mélange racémique.

4. Composé selon la revendication 2, pratiquement sous la forme d'un énantiomère unique.

5. Composé selon l'une quelconque des revendications 2 à 4, dans lequel le composé est le cis-2-hydroxyméthyl-5-(cytosin-1'-yl)-1,3-oxathiolane.

6. Composé selon l'une quelconque des revendications précédentes, pour l'utilisation dans la fabrication d'un médicament pour le traitement d'une infection virale.

7. Formulation pharmaceutique comprenant un composé selon l'une quelconque des revendications 2 à 5, associé à un véhicule pharmaceutiquement acceptable.

8. Formulation pharmaceutique selon la revendication 7, comprenant en plus, un autre agent thérapeutique.

9. Procédé de préparation d'un composé selon l'une quelconque des revendications 2 à 5, qui comprend :
(a) la réaction d'un composé de formule (VIII) 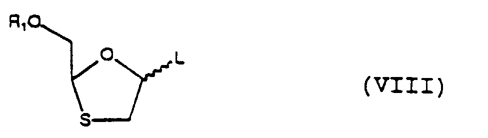 dans laquelle R₁ est un atome d'hydrogène, ou un groupe hydroxyle protecteur, et L est un atome ou un groupe déplaçable par la cytosine;
(b) l'interconversion basique du composé de formule (I') 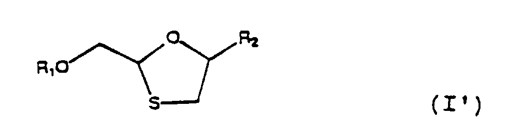 dans laquelle R₂ est une base purine ou pyrimidine autre que la cytosine , en cis-2-hydroxyméthyl-5-(cytosin-1'-yl)-1,3-oxathiolane ;
(c) la réaction d'un composé de formule (IX) 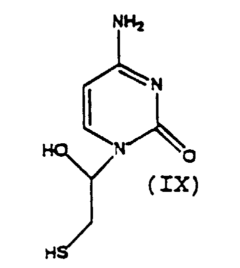 avec un composé de formule (X) 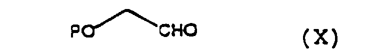 dans laquelle P est un groupe protecteur ; ou (d) la conversion d'un composé de formule (XII) 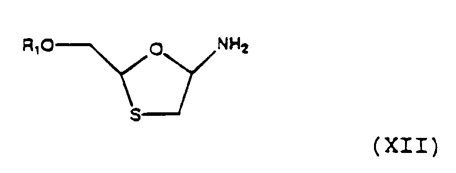 en cis-2-hydroxyméthyl-5-(cytosin-1'-yl)-1,3-oxathiolane,
et si nécessaire, ou désiré, la soumission du composé obtenu à l'une quelconque des étapes (a) à (d), à une ou deux autres réactions comprenant :
(i) le déplacement de tous les groupes protecteurs ;
(ii) la conversion du cis-2-hydroxyméthyl-5-(cytosin-1'-yl)-1,3-oxathiolane, ou d'un sel de celui-ci, en un sel de celui-ci, pharmaceutiquement acceptable.

10. Procédé selon la revendication 9, dans lequel le composé obtenu est sous la forme d'un mélange racémique.

11. Procédé selon la revendication 9, dans lequel le produit obtenu est pratiquement sous la forme d'un énantiomère unique.

12. Procédé selon l'une quelconque des revendications 9 à 11, dans lequel, à l'étape (a), le groupe L est choisi au sein d'un ensemble comprenant les groupes alcoxycarbonyle, l'iode, le brome, le chlore, ou le groupe -OR, où R est un groupe alkyle substitué ou non substitué, saturé ou non saturé, ou bien R est un groupe aliphatique substitué ou non substitué, ou un groupe acyle aromatique.

13. Procédé selon l'une quelconque des revendications 9 à 12, dans lequel, à l'étape (a), le composé de formule (VIII) est mis en réaction avec une cytosine silylée, dans un solvant compatible, en présence d'un acide de Lewis, ou de triméthylsilyltriflate.

14. Méthode de préparation d'une formulation pharmaceutique, comprenant le mélange d'un composé selon l'une quelconque des revendications 2 à 5, à un véhicule pharmaceutiquement acceptable.

## Revendications (Revendications pour l'(les) Etat(s) contractant(s) suivant(s): ES, GR)

1. Procédé de fabrication d'un cis-1.3-oxathiolane de formule : 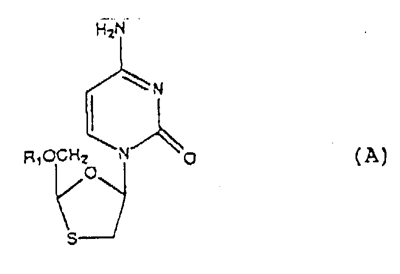 dans laquelle R₁ représente un atome d'hydrogène, un groupe alcanoyle C₁₋₁₆, un groupe benzoyle, ou un groupe benzoyle substitué par au moins un halogéne, un groupe alkyle C₁₋₆, alcoxy C_{1-6,} nitro, ou trifluorométhyle, ou d'un sel de celui-ci, pharmaceutiquement acceptable, sous la forme d'un isomère optique unique, ou d'un mélange d'isomères optiques, qui comprend :
(a) la réaction d'un composé de formule (VIII) 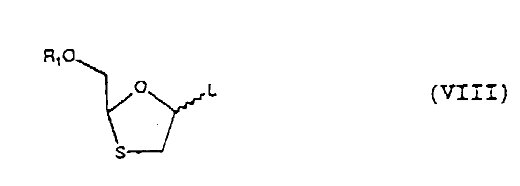 dans laquelle R₁ est un atome d'hydrogène, ou un groupe hydroxyle protecteur, et L est un atome ou un groupe déplaçable par la cytosine ;
(b) l'interconversion basique du composé de formule (I') 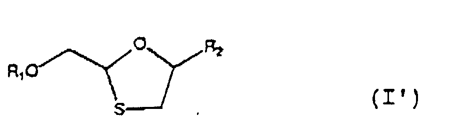 dans laquelle R₂ est une base purine ou pyrimidine autre que la cytosine, en cis-2-hydroxyméthyl-5-(cytosin-1'-yl)-1,3-oxathiolane ;
(c) la réaction d'un composé de formule (IX) 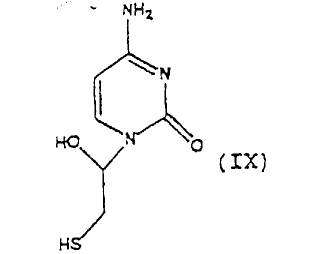 avec un composé de formule (X) 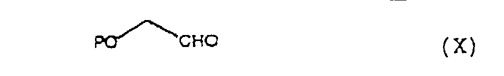 dans laquelle P est un groupe protecteur ; ou
(d) la conversion d'un composé de formule (XII) 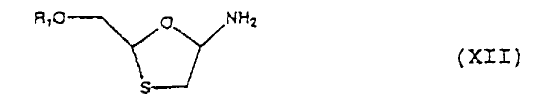 en cis-2-hydroxyméthyl-5-(cytosin-1'-yl)-1,3-oxathiolane,
et si nécessaire, ou désiré, la soumission du composé obtenu à l'une quelconque des étapes (a) à (d), à une ou deux autres réactions comprenant:
(i) le déplacement de tous les groupes protecteurs ;
(ii) la conversion du cis-2-hydroxyméthyl-5-(cytosin-1'-yl)-1,3-oxathiolane, ou d'un sel de celui-ci, en un sel de celui-ci, pharmaceutiquement acceptable.

2. Procédé selon la revendication 1, dans lequel le composé obtenu est le cis-2-hydroxyméthyl-5-(cytosin-1'-yl)-1,3.oxathio-ou un sel de celui-ci, pharmaceutiquement acceptable.

3. Procédé selon la revendication 2, dans lequel le composé obtenu est sous la forme d'un mélange racémique,

4. Procédé selon la revendication 2, dans lequel le composé obtenu est pratiquement sous la forme d'un énantiomère unique.

5. Procédé selon l'une quelconque des revendications précédentes, dans lequel le composé obtenu est le cis-2-hydroxyméthyl-5-(cytosin-1'-yl)-1,3-oxathiolane.

6. Procédé selon l'une quelconque des revendications 2 à 5, dans lequel, à l'étape (a), le groupe L est choisi au sein d'un ensemble comprenant les groupes alcoxycarbonyle, l'iode, le brome, le chlore, ou le groupe -OR, où R est un groupe alkyle substitué ou non substitué, saturé ou non saturé, ou bien R est un groupe aliphatique substitué ou non substitué, ou un groupe acyle aromatique.

7. Procédé selon l'une quelconque des revendications 2 à 6, dans lequel, à l'étape (a), le composé de formule (VIII) est mis en réaction avec une cytosine silylée, dans un solvant compatible, en présence d'un acide de Lewis, ou de triméthylsilyltriflate.
